(19) Europäisches Patentamt
European Patent Office
Office européen des brevets

(11) Veröffentlichungsnummer: 0 167 068
B1

(12) # EUROPÄISCHE PATENTSCHRIFT

(45) Veröffentlichungstag der Patentschrifft: 28.02.90

(51) Int. Cl.⁵: C 07 D 211/90, A 61 K 31/44

(21) Anmeldenummer: 85107622.4

(22) Anmeldetag: 20.06.85

(54) **1,4-Dihydro-2,6-dimethyl-4-(3-nitrophenyl)-pyridin-3,5-dicarbonsäure-isopropyl- 2-(3-trifluormethylphenoxy)-ethyl · ester, mehrere Verfahren zu seiner Herstellung sowie seine Verwendung in Arzneimitteln.**

(30) Priorität: 03.07.84 DE 3424342

(43) Veröffentlichungstag der Anmeldung: 08.01.86 Patentblatt 86/02

(45) Bekanntmachung des Hinweises auf die Patenterteilung: 28.02.90 Patentblatt 90/09

(84) Bennante Vertragsstaaten: AT BE CH DE FR GB IT LI NL SE

(56) Entgegenhaltungen:
EP-A-0 088 938
DE-A-2 508 181

**Die Akte enthält technische Angaben, die nach dem Eingang der Anmeldung eingereicht wurden und die nicht in dieser Patentschrift enthalten sind.**

(73) Patentinhaber: BAYER AG
D-5090 Leverkusen 1 Bayerwerk (DE)

(72) Erfinder: Wehinger, Egbert, Dr.
Gellertweg 33
D-5600 Wuppertal 1 (DE)
Erfinder: Knorr, Andreas, Dr.
Pahlkestrasse 15
D-5600 Wuppertal 1 (DE)
Erfinder: Stoepel, Kurt, Dr.
In den Birken 69
D-5600 Wuppertal 1 (DE)
Erfinder: Heise, Arend, Dr.
Egelser Strasse 5
D-2960 Aurich (DE)

EP 0 167 068 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBERGRAF, STOCKHOLM 1990

## Beschreibung

Die vorliegende Erfindung betrifft den 1,4-Dihydro-2,6-dimethyl-4-(3-nitrophenyl)-pyridin-3,5-dicarbonsäure-isopropyl-[2-(3-trifluormethylphenoxy)-ethyl]-ester, mehrere Verfahren zu seiner Herstellung sowie seine Verwendung in Arzneimitteln.

Es ist bekannt, daß bestimmte 1,4-Dihydropyridine interessante pharmakologische Eigenschaften aufweisen (F. Bossert und W. Vater, Naturwissenschaften *58*, 578(1971)).

In den älteren Patentanmeldungen EP-A 088 938 und DE-A 2 508 181 werden bereits Dihydropyridinverbindungen mit ähnlicher Struktur als Wirkstoffe und/oder Zwischenprodukte zur Herstellung von Wirkstoffen beschrieben.

Es wurde gefunden, daß der 1,4-Dihydro-2,6-dimethyl-4-(3-nitrophenyl)-pyridin-3,5-dicarbonsäure-isopropyl-[ 2-(3-trifluormethylphenoxy)-ethyl ]-ester der Formel I

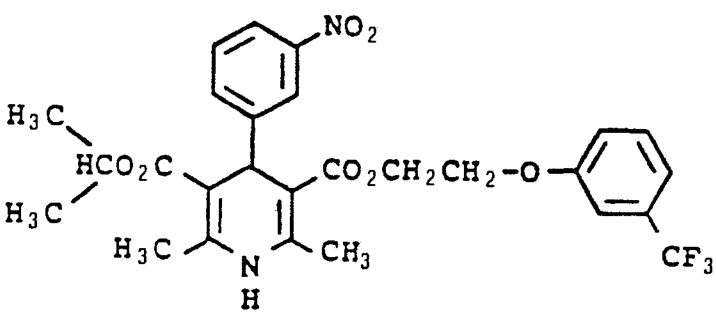

I

bevorzugt und langanhaltend peripher-vasodilatierend wirksam ist und somit als Wirkstoff in Arzneimitteln eingesetzt werden kann.

Weiterhin wurde gefunden, daß man den neuen Wirkstoff der Formel 5 erhalt, wenn man

A) 2-(3-Nitrobenzyliden)-acetessigsäureisopropylester der Formel II

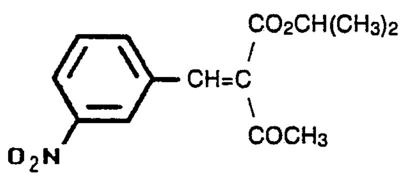

II

mit 3-Aminocrotonsäure-[2-3-trifluormethylphenoxy)-ethyl]-ester der Formel III

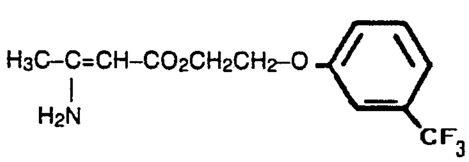

III

als solche oder in Gegenwart von Wasser und/oder inerten organischen Lösungsmitteln umsetzt,
oder

B) 2-(3-Nitrobenzyliden)-acetessigsäureisopropylester der Formel II mit Acetessigsäure-[2-(3-trifluormethylphenoxy)-ethyl]-ester der Formel IV

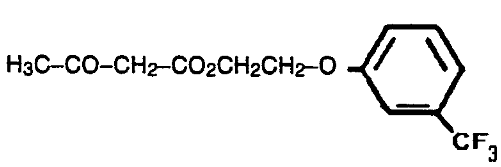

IV

und Ammoniak als solche oder in Gegenwart von Wasser und/oder inerten organischen Lösungsmitteln umsetzt,
oder

C) 2-(3-Nitrobenzyliden)-acetessigsäure-[2-(3-trifluormethylphenoxy)-ethyl]-ester der Formel VI

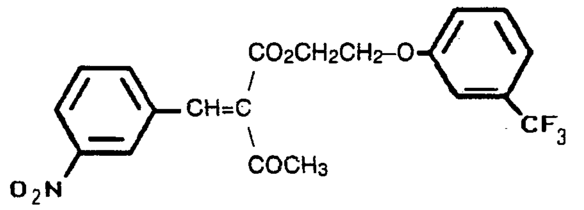

V

mit 3-Aminocrotonsäureisopropylester der Formel VI

$H_3C-C=CH-CO_2CH(CH_3)_2$ VI

|

$NH_2$

als solche oder in Gegenwart von Wasser und/oder inerten organischen Lösungsmitteln umsetzt,
oder

D) 2-(3-Nitrobenzylinden)-acetessigsäure-[2-(3-trifluormethylphenoxy)-ethyl]-ester der Formel V mit Acetessigsäureisoproplyester der Formel VII

$H_3C-CO-CH_2-CO_2CH(CH_3)_2$ VII

und Ammoniak als solche oder in Gegenwart von Wasser und/oder inerten organischen Lösungsmitteln umsetzt,
oder

E) 3-Nitrobenzaldehyd der Formel VIII

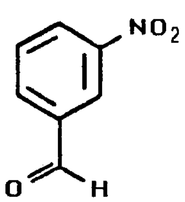

VIII

mit Acetessigsäure-[2-(3-trifluormethylphenoxy)-ethyl]-ester der Formel IV und 3-Aminocrotonsäureisopropyl-ester der Formel VI als solche oder in Gegenwart von Wasser und/oder inerten organischen Lösungsmitteln umsetzt,
oder

F) 3-Nitrobenzaldehyd der Formel VIII mit 3-Aminocrotonsäure-[2-(3-trifluormethylphenoxy)-ethyl]-ester der Formel III und Acetessigsäureisopropylester der Formel VII als solche oder in Gegenwart von Wasser und/oder inerten organischen Lösungsmitteln umsetzt.

Überraschenderweise ist die erfindungsgemäße Substanz der Formel I bevorzugt und langanhaltend peripher vasodilatierend wirksam.

In der Reihe der aus dem Stand der Technik bekannten ähnlichen 1,4-Dihydropyridin-Derivate ist bisher eine derartig bevorzugte und langanhaltende periphere Vasodilatation nicht gefunden worden, so daß die erfindungsgemäße Verbindung hinsichtlich dieser speziellen Eigenschaft eine Bereicherung der Pharmazie darstellt.

Die erfindungsgemäße Verbindung ist chiral und kann in stereoisomeren Formen existieren, die sich wie Bild und Spiegelbild verhalten (Enantiomere, Antipoden). Diese können ihrerseits wieder in verschiedenen Konformationen auftreten. Sowohl die Racemform als auch die Antipoden sind Gegenstand der vorliegenden Erfindung.

Je nach der Art der verwendeten Ausgangsstoffe kann die Synthese der erfindungsgemäßen Verbindung durch folgende Formelschemata wiedergegeben werden:

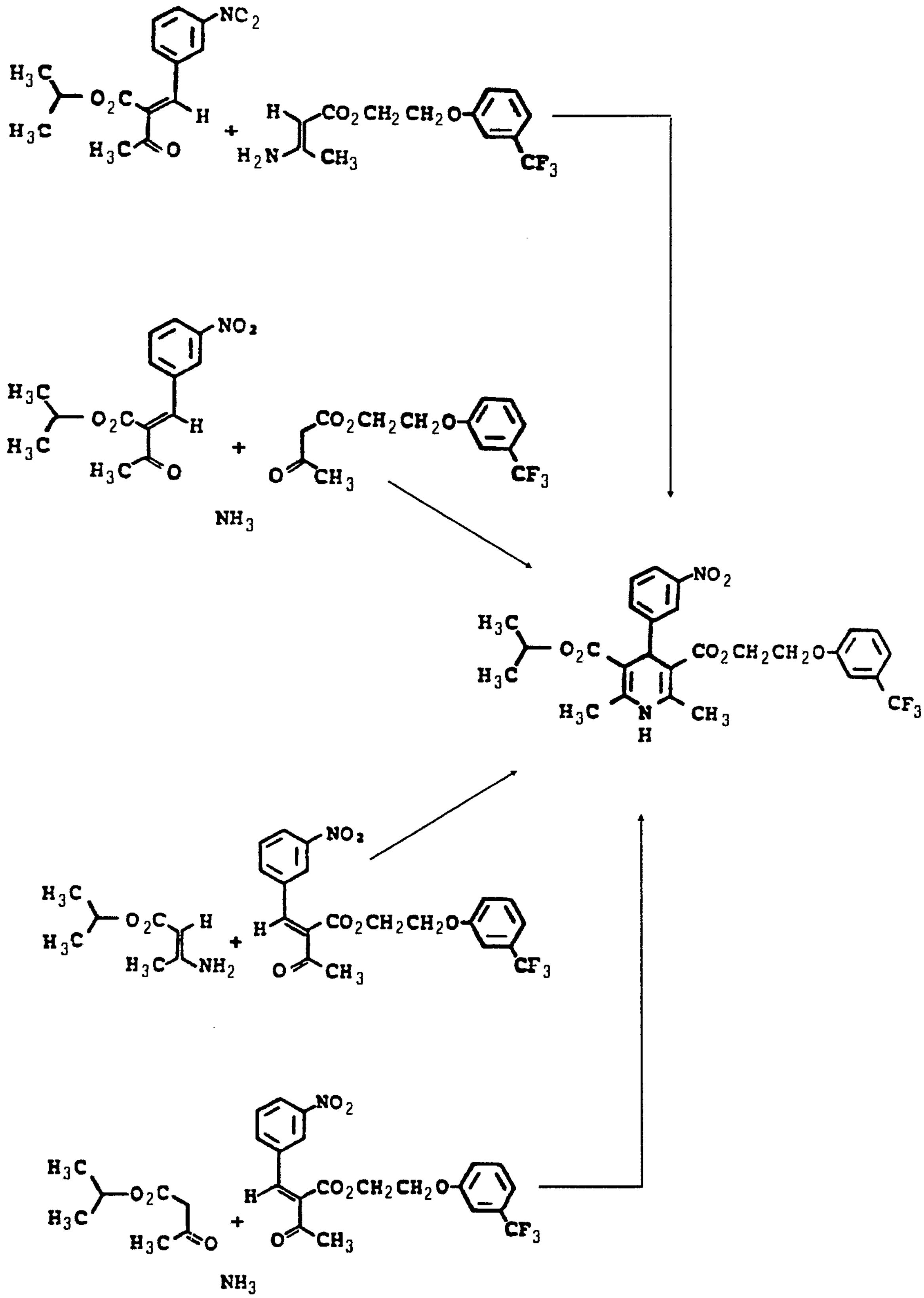

A)

B)

C)

D)

E)

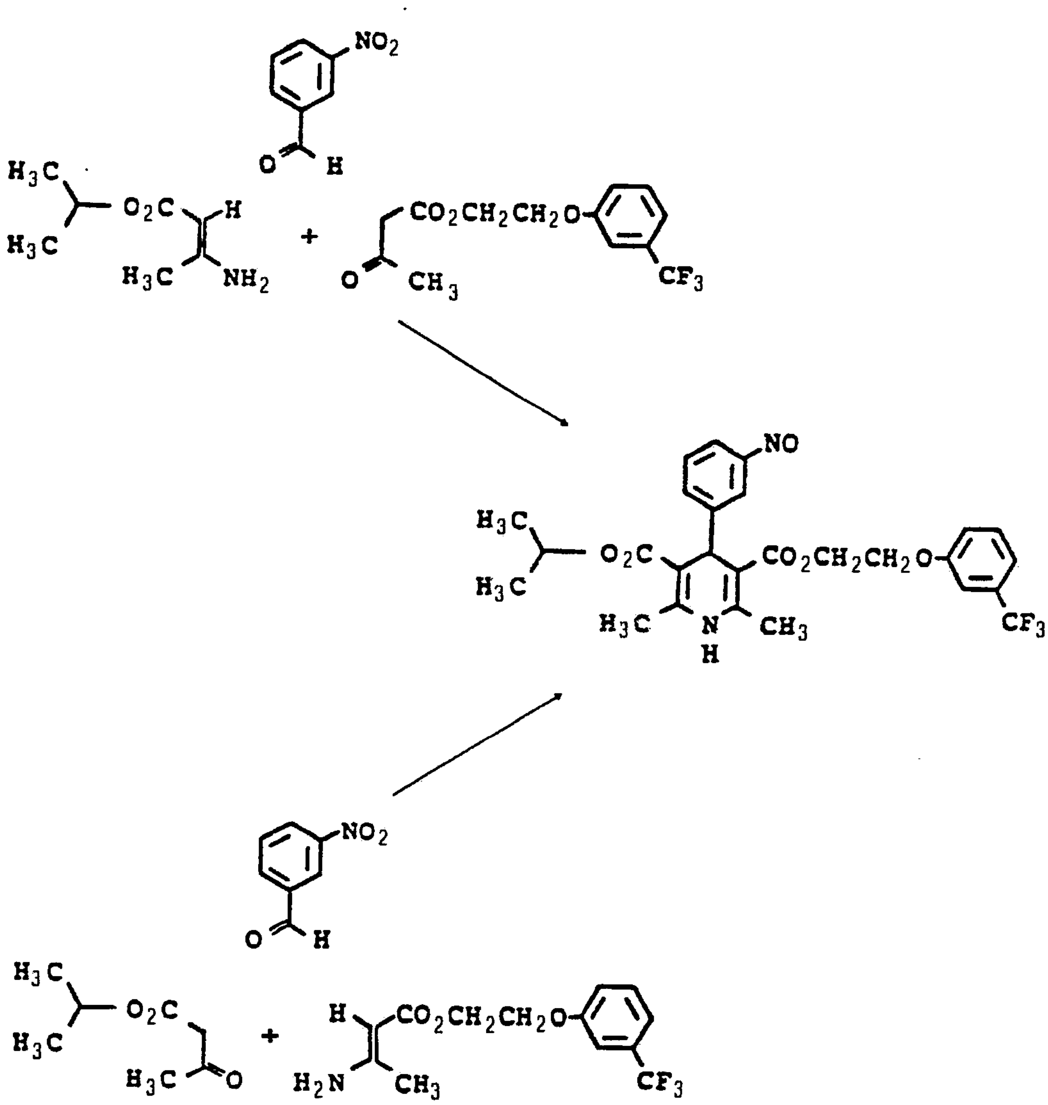

F)

Die als Ausgangsstoffe verwendeten Substanzen der Formeln II bis VIII sind literaturbekannt oder können nach literaturbekannten Methoden hergestellt werden (s. z. B. G. Jones "The Knoevenagel Condensation" in org. Reactions, Vol. XV, 204 ff (1967); A.C. Cope, J. Amer. Chem. Soc. *67*, 1017 (1945); D. Borrmann, "Umsetzung von Diketen mit Alkoholen, Phenolen und Mercaptanen" in Houben-Weyl, Methoden der Organischen Chemie, Vol. VII/4, 230 ff (1968)).

Bei der Durchführung der erfindungsgemäßen Verfahren A - F werden die an der Reaktion beteiligten Stoffe jeweils etwa in molaren Mengen eingesetzt. Das verwendete Ammoniak wird zweckmäßig in Überschuß zugegeben und zwar bevorzugt in 1,5 - 2,5 molaren Mengen, bezogen auf jeweils 1 Mol eines anderen Ausgangsstoffes.

Als Lösungsmittel kommen Wasser und alle inerten organischen Lösungsmittel in Frage. Dazu gehören vorzugsweise Alkohole wie Ethanol, Methanol oder Propanol oder Ether wie Diethylether, Tetrahydrofuran oder Dioxan oder Eisessig, Pyridin, Dimethylformamid, Dimethylsulfoxid, Acetonitril oder Hexamethylphosphorsäuretriamid.

Die Reaktionstemperaturen können in einem größeren Bereich variiert werden. Im allgemeinen arbeitet man etwa zwischen 20°C und 200°C, vorzugsweise bei 50°C bis 120°C oder insbesondere bei der Siedetemperatur des jeweiligen Lösungsmittels.

Die Umsetzung kann bei Normaldruck, aber auch bei erhöhtem Druck durchgeführt werden. Im allgemeinen arbeitet man bei Normaldruck.

Die vorstehenden Herstellungsverfahren sind lediglich zur Verdeutlichung angegeben und die Herstellung der Verbindung I ist nicht auf diese Verfahren beschränkt, sondern jede Modifikation dieser Verfahren ist in gleicher Weise für die Herstellung der Verbindung I anwendbar.

Bevorzugt genannt seien die erfindungsgemäßen Herstellungsverfahren A und C.

Die erfindungsgemäße Verbindung ist eine als Arzneimittel verwendbare Substanz. Sie bewirkt bei enteraler und parenteraler Anwendung eine starke und lang anhaltende periphere Vasodilatation und kann daher zur Therapie und Prophylaxe des Bluthochdruckes und/oder peripherer Durchblutungsstörungen eingesetzt werden. Die Verbindung kann daher zur Bekämpfung von Erkrankungen eingesetzt werden.

Der neue Wirkstoff kann in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nichttoxischer, pharmazeutisch geeigneter Trägerstoffe oder Lösungsmitte1. Hierbei soll die therapeutisch wirksame Verbindung jeweils in einer Konzentration von etwa 0,5 bis 90 Gewichtsprozent der Gesamtmischung vorhanden sein, d. h. in Mengen, die ausreichend sind, um den angegebenen Dosierungsspielraum zu erreichen.

Die Formulierungen werden beispielsweise hergestellt durch Verstrecken des Wirkstoffs mit Lösungsmitteln und/oder Trägerstoffen, gegebenenfalls unter Verwendung von Emulgiermitteln und/oder Dispergiermitteln, wobei z. B. im Fall der Benutzung von Wasser als Verdünnungsmittel gegebenenfalls organische Lösungsmittel als Hilfslösungsmittel verwendet werden können.

Als Hilfsstoffe seien beispielhaft aufgeführt:

Wasser, nichttoxische organische Lösungsmittel, wie Paraffine (z. B. Erdölfraktionen), pflanzliche Öle (z. B. Erdnuß-Sesamöl), Alkohole (z. B. Ethylalkohol, Glycerin), Glykole (z. B. Propylenglykol, Polyethylenglykol); feste Trägerstoffe, wie z. B. natürliche Gesteinsmehle (z. B. Kaoline, Tonerden, Talkum, Kreide), synthetische Gesteinsmehle (z. B. hochdisperse Kieselsäure, Silikate), Zucker (z. B. Rohr-, Milch- und Traubenzucker); Emulgiermittel, wie nichtionogene und anionische Emulgatoren (z. B. Polyoxyethylen-Fettsäure-Ester, Polyoxyethylen-Fettalkohol-Ether, Alkylsulfonate und Arylsulfonate), Dispergiermittel (z. B. Lignin, Sulfitablaugen, Methylcellulose, Stärke und Polyvinylpyrrolidon) und Gleitmittel (z. B. Magnesiumstearat, Talkum, Stearinsäure und Natriumlaurylsulfat).

Die Applikation erfolgt in üblicher Weise, vorzugsweise enteral oder parenteral, insbesondere oral oder intravenös.

Im Falle der enteralen Anwendung können Tabletten selbstverständlich außer den genannten Trägerstoffen auch Zusätze wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin könne Gleitmittel wie Magnesiumstearat, Natriumlaurylsulfat und Talkum zum Tabletteieren mitverwendet werden. Im Falle wäßringer Suspensionen und/oder Elixieren, die für orale Anwendungen gedacht sind, können die Wirkstoffe außer mit den obengenannten Hilfsstoffen mit verschiedenen Geschmacksaufbessern oder Farbstoffen versetzt werden.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,0001 bis 1 mg/kg, vorzugsweise etwa 0,0014 bis 0,10 mg/kg Körpergewicht pro Tag zur Erzielung wirksamer Ergebnisse zu verabreichen, und bei enteraler Applikation beträgt die Dosierung etwa 0,01 bis 10 mg/kg, vorzugsweise 0,1 bis 1,0 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch auf Grund der Tierart und deren individuellem Verhalten gegenüber dem Medikament bzw. Intervall, zu welchem die Verabreichung erfolgt.

So kann es in einigen Fällen ausreichend sein, mit weniger als der vorgenannten Mindestmenge auszukommen, während in anderen Fällen die genannte obere Grenze überschritten werden muß. Im Fall der Applikation größerer Mengen kann es empfehlenswert sein, diese in mehrere Einzelgaben über den Tag zu verteilen.

Für die Applikation in der Humanmedizin ist der gleiche Dosierungsspielraum vorgesehen. Sinngemäß gelten hierbei auch die obigen Ausführungen.

**Herstellungsbeispiele**

1,4-Dihydro-2,6-dimethyl-4-(3-nitrophenyl)-pyridin-3,5-dicarbonsäure-isoproply-[2-(3-trifluormethylphenoxy)-ethyl]-ester

Figur

A) Eine Lösung von 27,7 g (0,1 Mol) 2-(3-Nitrobenzyliden)-acetessigsäureisopropylester und 28,9 g (0,1 Mol) 3-Aminocrotonsäure-[2-(3-trifluormethylphenoxy)-ethyl]-ester in 150 ml Ethanol wurde 20 Stunden unter Stickstoff zum Sieden erhitzt. Anschließend wurde das Lösungsmittel im Vakuum abdestilliert und der ölige Rückstand durch Verreiben mit wenig Ether zur Kristallisation gebracht. Das Rohprodukt wurde abgesaugt und aus Ethanol umkristallisiert.
Schmelzpunkt: 128 - 130 °C
Ausbeute: 35 g (64 %).

B) 27,7 g (0,1 Mol) 2-(3-Nitrobenzyliden)-acetessigsäureisopropylester wurden zusammen mit 29 g (0,1 Mol) Acetessigsäure-[2-(3-trifluormethylphenoxy)-ethyl]-ester und 12 g (0,18 Mol) einer 25 % wäßrigen Ammoniaklösung in 160 ml Isopropanol 24 Stunden in einer Stickstoffatmosphäre unter Rückfluß erhitzt. Anschließend wurde das Lösungsmittel im Vakuum abdestilliert und der ölige Rückstand zur Kristallisation gebracht. Das Rohprodukt wurde abgesaugt und aus Ethanol umkristallisiert.
Schmelzpunkt: 128 - 130 °C
Ausbeute: 26 g (47 %).

C) 42.3 g (0,1 Mol) 2-(3-Nitrobenzyliden)-acetessigsäure-[2-(3-trifluormethylphenoxy)-ethyl]-ester wurden zusammen mit 14.3 g (0,1 Mol) 3-Aminocrotonsäureisopropylester in 150 ml Ethanol 20 Stunden unter Stickstoff unter Rückfluß erhitzt. Anschließend wurde das Lösungsmittel im Vakuum abdestilliert und der ölige Rückstand mit wenig Ether durchmischt. Nach kurzer Zeit trat Kristallisation ein. Das Rohprodukt wurde abgesaugt und aus Ethanol umkristallisiert.
Schmelzpunkt: 128 - 130 °C
Ausbeute: 31 g (57 %)

D) 42,3 g (0,1 Mol) 2-(3-Nitrobenzyliden)-acetessigsäure-[2-(3-trifluormethylphenoxy)-ethyl]-ester wurden zusammen mit 14,4 g (0,1 Mol) Acetessigsäureisopropylester und 12 g (0,18 Mol) einer 25 % wäßrigen Ammoniaklösung in 150 ml Isopropanol 24 Stunden in einer Sickstoffatmosphäre unter Rückfluß erhitzt. Nach dem Erkalten der Reaktionsmischung wurde das Lösungsmittel im Vakuum abdestilliert und der ölige Rückstand in Dichlormethan aufgenommen. Die organische Phase wurde mit Wasser gewaschen und nach Trocknen über wasserfreiem Natriumsulfat im Vakuum eingeengt. Das resultierende Öl wurde zur Kristallisation gebracht, das Rohprodukt abgesaugt und aus Ethanol umkristallisiert.
Schmelzpunkt: 128 - 130 °C
Ausbeute: 28 g (51 %).

E) Eine Lösung von 15,1 g (0,1 Mol) 3-Nitrobenzaldehyd, 29 g (0,1 Mol) Acetessigsäure-[2-(3-trifluormethylphenoxy)-ethyl]-ester und 14,3 g (0,1 Mol) 3-Aminocrotonsäureisopropylester in 150 ml Dioxan wurde 24 Stunden unter Stickstoff zum Sieden erhitzt. Anschließend wurde das Lösungsmittel im Vakuum abdestilliert und der ölige Rückstand durch Verreiben mit wenig Ether zur Kristallisation gebracht. Der Feststoff wurde abgesaugt und aus Ethanol umkristallisiert.
Schmelzpunkt: 128 - 130 °C
Ausbeute: 25 g (46 %).

F) Eine Lösung von 15,1 g (0,1 Mol) 3-Nitrobenzaldehyd, 28,9 g (0,1 Mol) 3-Aminocrotonsäure-[2-(3-trifluormethylphenoxy)-ethyl]-ester und 14,4 g (0,1 Mol) Acetessigsäureisopropylester in 150 ml Isopropanol wurde 24 Stunden unter Stickstoff zum Sieden erhitzt. Nach Erkalten der Reaktionsmischung wurde das Lösungsmittel im Vakuum abdestilliert, der Rückstand mit Ether verrieben, das kristallisierte Produkt abgesaugt und aus Ethanol umkristallisiert.
Schmelzpunkt: 128 - 130 °C
Ausbeute: 24 g (44 %).

**Patentansprüche**

1. 1,4-Dihydro-2,6-dimethyl-4-(3-nitrophenyl)-pyridin-3,5-dicarbonsäure-isoproply-[2-(3-trifluormethylphenoxy)-ethyl]-ester der Formel I

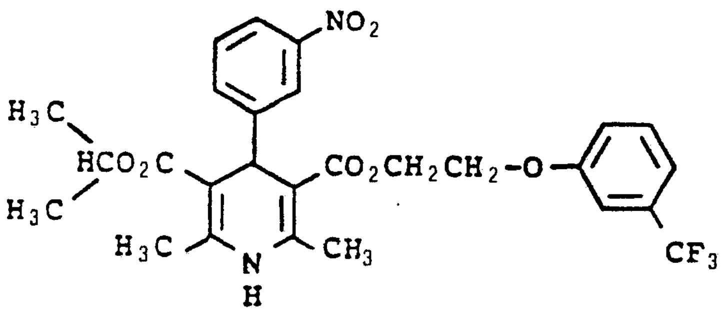

I

2. 1,4-Dihydro-2,6-dimethyl-4-(3-nitrophenyl)-pyridin-3,5-dicarbonsäure-isoproply-[2-(3-trifluormethylphenoxy)-ethyl]-ester zur Bekämpfung von Erkrankungen.

3. 1,4-Dihydro-2,6-dimethyl-4-(3-nitrophenyl)-pyridin-3,5-dicarbonsäure-isopropyl-[2-(3-trifluormethylphenoxy)-ethyl]-ester zur Bekämpfung von Bluthochdruck und/oder peripherer Durchblutungsstörungen.

4. Arzneimittel enthaltend als Wirkstoff 1,4-Dihydro-2,6-dimethyl-4-(3-nitrophenyl)-pyridin-3,5-dicarbonsäure-isoproply-[2-(3-trifluormethylphenoxy)-ethyl]-ester.

5. Verfahren zur Herstellung eines Arzneimittels enthaltend 1,4-Dihydro-2,6-dimethyl-4-(3-nitrophenyl)-pyridin-3,5-dicarbonsäure-isoproply-[2-(3-trifluormethyl-phenoxy)-ethyl]-ester, dadurch gekennzeichnet, daß man den Wirkstoff mit üblichen Hilfs- und/oder Trägerstoffen mischt.

6. Verwendung des 1,4-Dihydro-2,6-dimethyl-4-(3-nitrophenyl)-pyridin-3,5-dicarbonsäure-isopropyl-[2-(3-trifluormethyl-

phenoxy)-ethyl]-ester zur Herstellung von Arzneimitteln zur Bekämpfung von Bluthochdruck und/oder peripherer Durchblutungsstörungen.

7. Verfahren zur Herstellung des 1,4-Dihydro-2,6-dimethyl-4-(3-nitrophenyl)-pyridin-3,5-dicarbonsäureisopropyl-[2-(3-trifluormethylphenoxy)-ethyl]-ester, dadurch gekennzeichnet, daß man

A) 2-(3-Nitrobenzyliden)-acetessigsäureisopropylester der Formel II

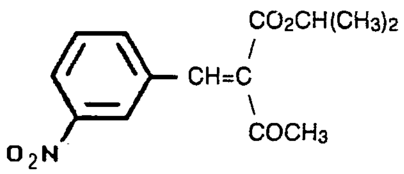

II

mit 3-Aminocrotonsäure-[2-(3-trifluormethylphenoxy)-ethyl]-ester der Formel III

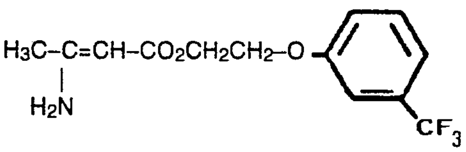

III

als solche oder in Gegenwart von Wasser und/oder inerten organischen Lösungsmitteln umsetzt,
oder

B) 2-(3-Nitrobenzyliden)-acetessigsäureisopropylester der Formel II mit Acetessigsäure-[2-(3-trifluormethylphenoxy)-ethyl]-ester der Formel IV

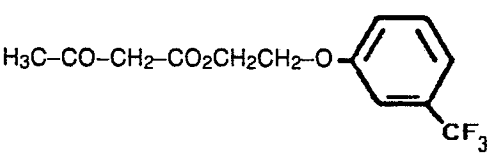

IV

und Ammoniak als solche oder in Gegenwart von Wasser und/oder inerten organischen Lösungsmitteln umsetzt,
oder

C) 2-(3-Nitrobenzyliden)-acetessigsäure-[2-(3-trifluormethylphenoxy)-ethyl]-ester der Formel V

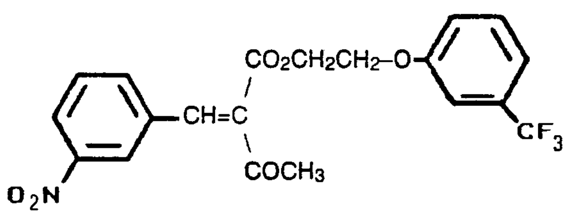

V

mit 3-Aminocrotonsäureisopropylester der Formel VI

$H_3C-C=CH-CO_2CH(CH_3)_2$ VI
$\;\;\;\;|$
$\;\;\;NH_2$

als solche oder in Gegenwart von Wasser und/oder inerten organischen Lösungsmitteln umsetzt,
oder

D) 2-(3-Nitrobenzyliden)-acetessigsäure-[2-(3-trifluormethylphenoxy)-ethyl]-ester der Formel V mit Acetessigsäureisopropylester der Formel VII

$H_3C\text{-}CO\text{-}CH_2\text{-}CO_2CH(CH_3)_2$ VII

und Ammoniak als solche oder in Gegenwart von Wasser und/oder inerten organischen Lösungsmitteln umsetzt, oder

E) 3-Nitrobenzaldehyd der Formel VIII

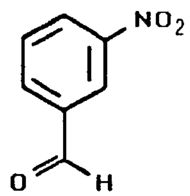

VIII

mit Acetessigsäure-[2-(3-trifluormethylphenoxy)-ethyl]-ester der Formel IV und 3-Aminocrotonsäureisopropylester der Formel VI als solche oder in Gegenwart von Wasser und/oder inerten organischen Lösungsmitteln umsetzt, oder

F) 3-Nitrobenzaldehyd der Formel VIII mit 3-Aminocrotonsäure-[2-(3-trifluormethylphenoxy)-ethyl]-ester der Formel III und Acetessigsäureisopropylester der Formel VII als solche oder in Gegenwart von Wasser und/oder inerten organischen Lösungsmitteln umsetzt.

8. Verfahren gemäß Anspruch 8, dadurch gekennzeichnet, daß die Herstellungsvarianten A oder C benutzt werden.

9. Verfahren gemäß Ansprüchen 8 und 9, dadurch gekennzeichnet, daß die Umsetzung bei Temperaturen zwischen 20 und 200 °C durchgeführt wird.

**Claims**

1. Isopropyl 2-(3-trifluoromethylphenoxy)-ethyl 1,4-dihydro-2,6-dimethyl-4-(3-nitrophenyl)-pyridine-3,5-dicarboxylate of the formula I

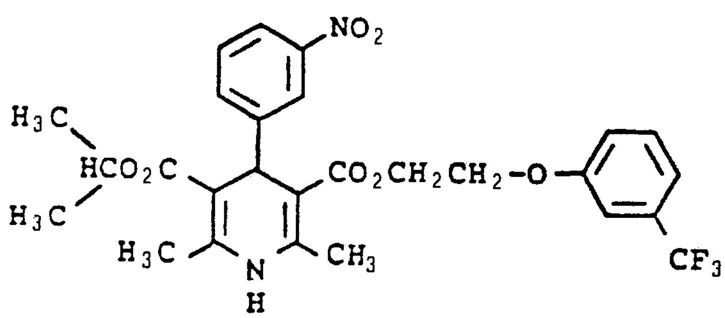

I

2. Isopropyl 2-(3-trifluoromethylphenoxy)-ethyl 1,4-dihydro-2,6-dimethyl-4-(3-nitrophenyl)-pyridine-3,5-dicarboxylate for combating diseases.

3. Isopropyl 2-(3-trifluoromethylphenoxy)-ethyl 1,4-dihydro-2,6-dimethyl-4-(3-nitrophenyl)-pyridine-3,5-dicarboxylate for combating hypertension and/or peripheral circulatory disturbances.

4. Medicaments containing, as the active compound, isopropyl 2-(3-trifluoromethylphenoxy)-ethyl 1,4-dihydro-2,6-dimethyl-4-(3-nitrophenyl)-pyridine-3,5-dicarboxylate.

5. Process for the preparation of a medicament containing isopropyl 2-(3-trifluoromethylphenoxy)-ethyl 1,4-dihydro-2,6-dimethyl-4-(3-nitrophenyl)-pyridine-3,5-dicarboxylate, characterised in that the active compound is mixed with customary auxiliaries and/or excipients.

6. Use of isopropyl 2-(3-trifluoromethylphenoxy)-ethyl 1,4-dihydro-2,6-dimethyl-4-(3-nitrophenyl)-pyridine-3,5-dicarboxylate for the preparation of medicaments for combating hypertension and/or peripheral circulatory disturbances.

7. Process for the preparation of isopropyl 2-(3-trifluoromethylphenoxy)-ethyl 1,4-dihydro-2,6-dimethyl-4-(3-nitrophenyl)-pyridine-3,5-dicarboxylate, characterised in that

A) isopropyl 2-(3-nitrobenzylidene)-acetoacetate of the formula II

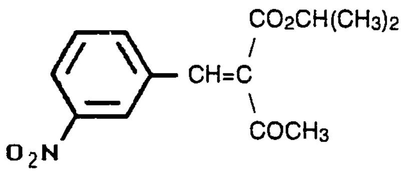

II

is reacted with 2-(3-trifluoromethylphenoxy)-ethyl 3-aminocrotonate of the formula III

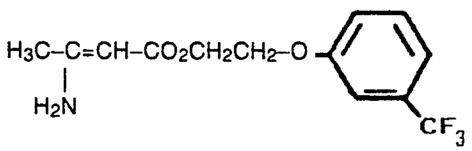

III

as such or in the presence of water and/or inert organic solvents, or

B) isopropyl 2-(3-nitrobenzylidene)-acetoacetate of the formula II is reacted with 2-(3-trifluoromethylphenoxy)-ethyl acetoacetate of the formula IV

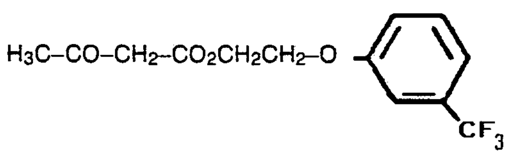

IV

and ammonia, as such or in the presence of water and/or inert organic solvents, or

C) 2-(3-trifluoromethylphenoxy)-ethyl 2-(3-nitrobenzylidene)-acetoacetate of the formula V

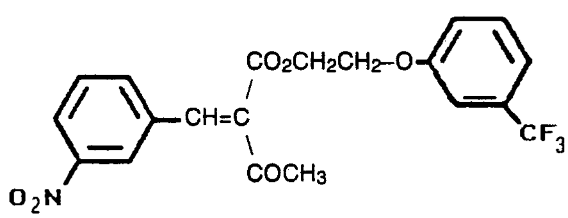

V

is reacted with isopropyl 3-aminocrotonate of the formula VI

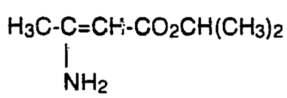

VI

as such or in the presence of water and/or inert organic solvents, or

D) 2-(3-trifluoromethylphenoxy)-ethyl 2-(3-nitrobenzylidene)-acetoacetate of the formula V is reacted with isopropyl acetoacetate of the formula VII

$H_3C\text{-}CO\text{-}CH_2\text{-}CO_2CH(CH_3)_2$ VII

and ammonia, as such or in the presence of water and/or inert organic solvents, or

E) 3-nitrobenzaldehyde of the formula VIII

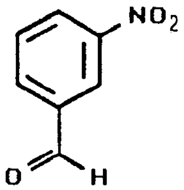

VIII

is reacted with 2-(3-trifluoromethylphenoxy)-ethyl acetoacetate of the formula IV and isopropyl 3-aminocrotonate of the formula VI, as such or in the presence of water and/or inert organic solvents, or

F) 3-nitrobenzaldehyde of the formula VIII is reacted with 2-(3-trifluoromethylphenoxy)-ethyl 3-aminocrotonate of the formula III and isopropyl acetoacetate of the formula VII, as such or in the presence of water and/or inert organic solvents.

8. Process according to claim 7, characterised in that process variants A or C are used.

9. Process according to claims 7 and 8, characterised in that the reaction is carried out at temperatures between 20 and 200 °C.

**Revendications**

1. L'ester d'isopropyle et de 2-(3-trifluorométhylphénoxy)-éthyle de l'acide 1,4-dihydro-2,6-diméthyl-4-(3-nitrophényl)-pyridine-3,5-dicarboxylique de formule I

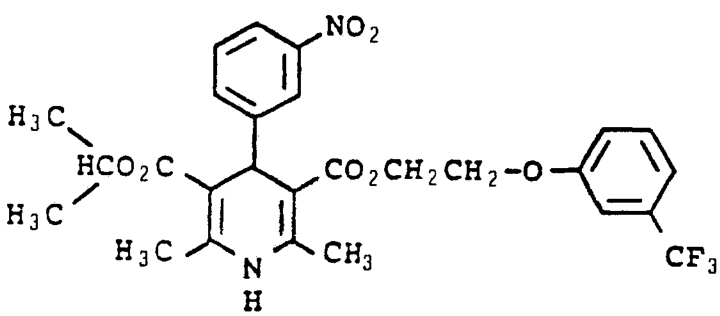

I

2. L'ester d'isopropyle et de 2-(3-trifluorométhylphénoxy)-éthyle de l'acide 1,4-dihydro-2,6-diméthyl-4-(3-nitrophényl)-pyridine-3,5-dicarboxylique, destiné à combattre des maladies.

3. L'ester d'isopropyle et de 2-(3-trifluorométhylphénoxy)-éthyle de l'acide 1,4-dihydro-2,6-diméthyl4-(3-nitrophényl)-pyridine-3,5-dicarboxylique, destiné à combattre un excès de pression sanguine et/ou des troubles de la circulation périphérique.

4. Médicament contenant comme substance active l'ester d'isopropyle et de 2-(3-trifluorométhylphénoxy)-éthyle de l'acide 1,4-dihydro-2,6-diméthyl-4-(3-nitrophényl)-pyridine-3,5-dicarboxylique.

5. Procédé de préparation d'un médicament contenant l'ester d'isopropyle et de 2-(3-trifluorométhylphénoxy)-éthyle de l'acide 1,4-dihydro-2,6-diméthyl-4-(3nitrophényl)-pyridine-3,5-dicarboxylique, caractérisé en ce qu'on mélange la substance active avec des substances auxiliaires et/ou des supports classiques.

6. Utilisation de l'ester d'isopropyle et de 2-(3-trifluorométhylphénoxy)-éthyle de l'acide 1,4-dihydro-2,6-diméthyl-4-(3-nitrophényl)-pyridine-3,5-dicarboxylique pour la préparation de médicaments destinés à combattre un excès de pression sanguine et/ou des troubles de la circulation périphérique.

7. Procédé de préparation de l'ester d'isopropyle et de 2-(3-trifluorométhylphénoxy)-éthyle de l'acide 1,4-dihydro-2,6-diméthyl-4-(3-nitrophényl)-pyridine-3,5-dicarboxylique, caractérisé en ce que

A) on fait réagir l'ester d'isopropyle de l'acide 2-(3-nitrobenzylidène)-acétylacétique de formule II

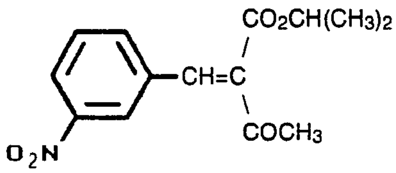

II

avec l'ester de 2-(3-trifluorométhylphénoxy)-éthyle de l'acide 3-aminocrotonique de formule III

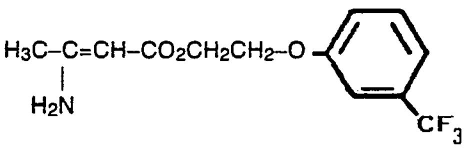

III

tels quels ou en présence d'eau et/ou de solvants organiques inertes, ou bien

B) on fait réagir l'ester d'isopropyle de l'acide 2-(3-nitrobenzylidène)-acétylacétique de formule II avec l'ester 2-(3-trifluorométhylphénoxy)-éthylique de l'acide acétylacétique de formule IV

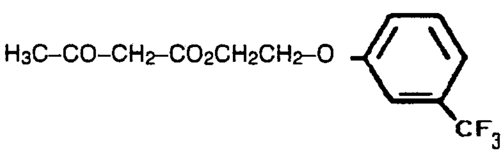

IV

et l'ammoniac tels quels ou en présence d'eau et/ou de solvants organiques inertes, ou bien

C) on fait réagir l'ester 2-(3-trifluorométhylphénoxy)-éthylique de l'acide 2-(3-nitrobenzylidène)-acétylacétique de formule V

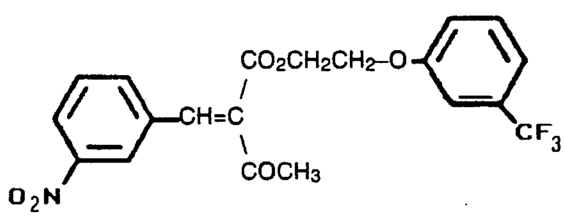

V

avec l'ester isopropylique de l'acide 3-aminocrotonique de formule VI

$H_3C-C=CH-CO_2-CH(CH_3)_2$ VI
|
$NH_2$

tels quels ou en présence d'eau et/ou de solvants organiques inertes, ou bien

D) on fait réagir l'ester 2-(3-trifluorométhylphénoxy)-éthylique de l'acide 2-(3-nitrobenzylidène)-acétylacétique de formule V avec l'ester isopropylique de l'acide acétylacétique de formule VII

$H_3C-CO-CH_2-CO_2CH(CH_3)_2$ VII

et l'ammoniac tels quels ou en présence d'eau et/ou de solvants organiques inertes, ou bien

E) on fait réagir le 3-nitrobenzaldéhyde de formule VIII

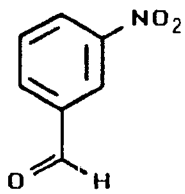

VIII

avec l'ester 2-(3-trifluorométhylphénoxy)-éthylique de l'acide acétylacétique de formule IV et l'ester isopropylique de l'acide 3-aminocrotonique de formule VI tels quels ou en présence d'eau et/ou de solvants organiques inertes, ou bien

F) on fait réagir le 3-nitrobenzaldéhyde de formule VIII avec l'ester 2-(3-trifluorométhylphénoxy)-éthylique de l'acide 3-aminocrotonique de formule III et l'ester isopropylique de l'acide acétylacétique de formule VII tels quels ou en présence d'eau et/ou de solvants organiques inertes.

8. Procédé suivant la revendication 7, caractérisé en ce qu'on utilise les variantes A ou C de préparation.

9. Procédé suivant les revendications 7 et 8, caractérisé en ce qu'on conduit la réaction à des températures comprises entre 20 et 200 °C.